# EUROPEAN PATENT APPLICATION

(11) **EP 1 820 507 A1**
(43) Date of publication of application: **22.08.2007**
(21) Application number: 06117523.8
(22) Date of filing: 19.07.2006
(51) Int. Cl.: A61K 35/74, A61K 31/715, A61K 9/20, A61K 9/28, C08B 37/00, A61P 13/08

(54) **A pharmaceutical composition for the medical treatment of the benign prostatic hyperplasia, its preparation method and its therapeutical application**

(30) Priority: 17.02.2006 AR P060100573
(71) Applicant: H.P.B., S. A., 1187 Buenos Aires (AR)
(72) Inventor: BARREIRO, Hipólito Carmelo Maria, 1187, BUENOS AIRES (AR)
(74) Representative: Barlocci, Anna

(57) **Abstract**

A solid pharmaceutical composition for the treatment of Benign Prostate Hyperplasia, a manufacturing procedure and the treatment method is provided in which the pharmaceutical composition at least includes between 0.5 and 5 mg of a polysaccharide extract from a Gram negative bacteria wall and also pharmaceutically acceptable excipients. The aforesaid bacteria are preferably *Pseudomonas Aeruginosa.* Excipients can be i.e. colloidal silicic anhydride, wheat starch, micro crystalline cellulose, lactose or a combination of all of them. The composition may be in form of pastilles, capsules, tablets or pills.

## Description

The present invention refers to a solid pharmaceutical composition for the treatment of the Benign Prostate Hyperplasia (BPH), its preparation procedure and therapeutic method, in which the pharmaceutical composition contains at least, between 0.5 and 5 mg of a Polysaccharide extract from the wall of gram negative bacteria and a pharmaceutically acceptable excipient. Said bacteria mainly are Pseudomonas Aeruginosa. The excipient may be e.g.: salicylic colloidal anhydrid, wheat starch, micro crystalline cellulose, lactose or a combination of all of them. The composition may be produced as pills, capsules, tablets or pellets.

### BACKGROUND OF THE INVENTION

### Benign Prostate Hyperplasia.

Benign Prostate Hyperplasia (BPH) is a common age related proliferate abnormality of the human prostate. Prostate tissue comprises 3 histological different zones: a) peripheral, b) central, c) transitional(TZ). The TZ normally takes up 5 % of the gland and is the only zone in which BPH develops (Mc Neal and collaborators J. Pathology of benign prostate hyperplasia. Insight into etiology. (Urol Clin North Am. 1990; 17: 477). An increase in the stromal compartment within the TZ tissue, leading to obstruction of urinary outflow, has been described as being the most prominent morphological difference between normal prostate and BPH tissue (Ishigooka M. et al. Prostate 1996; 29: 77).

Most likely, BPH is intrinsically a mesenchymal disease, resulting from a re-awakening of mensenchymal interactions between prostatic stroma and epithelium (Bierhoff E. The Prostate 1997; 31: 234-240). The stroma provides functionality and direction to prostate epithelial tissue.

Studies over recent years have identified intra-prostate signaling systems, acting as prostate transduction elements, are clearly important for the regulation of stromal cell proliferation and extra-cellular matrix production, the two major elements of prostate stroma. Strongly related to this is an apparent balance between polypeptide growth factors, including members of fibroblast grow factors (FGF), transforming grow factors (TGF) and connective tissue grow factors (CTGF). In BPH there is a lack of balance in the production of these three grow factors: FGF, TGF and CTGF. Over regulation of FGF generates increased prostatic cell proliferation. TGF- B1 over regulation stimulates collagen synthesis by inducing CTGF in stromal cells. Over expression of CTGF in BPH leads to excessive collagen accumulation, as seen in fibrotic disorders like Crohn's disease, systemic sclerosis or keloids.

There is a good evidence that above elements of this system are over expressed in BPH tissue. Potential modulators of the balance between these factors include senescence, estrogens, androgens, adrenergic signaling and inflammation, as causing BPH. Prostate inflammation is an extremely common histological finding in patients with symptoms of BPH who apparently have no symptoms of prostatitis (Nickel J.C. et al. BJU International 1999; 84: 976-981). Bacterial presence associated with BPH has been documented in patients with BPH (Hasner E et al. Acta Chir Scanda. 1962; 2:1 - 516). Hochreiter et al. proved a significant correlation between bacterial 16 S rDNA and BPH using a highly sensitive polymerase chain reaction (PCR) assay (Hochreiter W.W J Urol. 2000; 163 (1) : 127 - 30). These increased levels of bacterial 16 S ribosomal DNA are compatible with the notion that a bacterial inflammatory event initiates the mesenchymal re-awakening. Toll like receptors (TLR) recently identified, have been shown to play an important role in host cells responses toward bacteria, bacterial products, fungi and/or viruses. These receptors are found in the cells of different tissues, e.g.: intestinal mucous, prostate, innate immune system, etc. Different bacterial products mediate cellular activation via distinct TLR (Akira S. Toll-like receptor signaling. Nature Review Immunology 2004; 4: 499). Activation of TLR signaling pathways leads to the activation of genes of immune responses including the expression and release of various pro-inflammatory genes and chemotaxant cytokines.

Furthermore, bacterial products are capable of promoting fibrosis (Tol v. E.A.F. et al. Am. J. Physiol. 1999; 40: 277) Increased extra-cellular collagen synthesis and concomitant increased TGF- 1B and inter-leukin (IL)-6 expression was found by direct stimulation of mesenchymal cells. Neutralization of TGF-1B, inhibited *in vitro* collagen gene expression.

### Pseudomonas Aeruginosa Hapten.

Recently, an in-depth immunology study on BPH has brought to light the relationship between this disease with immunology mechanisms, specially the important intra-prostate signaling systems and the Toll like-receptors mentioned above, not so well-known so far outside of Immunology. Now perhaps, we can better understand the therapeutic possibility of PAH over BPH (Kuijpers K.A.J. Haptenotherapy for the treatment of benign prostate hyperplasia. Dept. of Exper. Urol. Niejmegen University and Medical Center. The Netherlands. March 2005).

A hapten is a non-immunogenic part unable of acting by itself. However, when attached to a lipid and protein carrier, it becomes an antigen, causing fever and toxic condition on one hand, but stimulating defense mechanisms on the other. Toxic effects and fever are originated in the lipid fraction. By obtaining in 1955 a lipid free Pseudomonas Aeruginosa Hapten (PAH), (Puebla LC et al. Treatment of allergic diseases with the Pseudomonas Aeruginosa Polysaccharide. IV SC.Sect.; 1955: aug.) it has been found that PAH lack toxic and only acted as a desensing immunomodulator. Pseudomonas Aeruginosa (PA) is a Gram-negative bacteria; unlike Gram-positive, have lipopolysaccharides(LPS) embedded in their outer cell wall layer. These LPS are recognized by the TOLL-like receptors of the innate immune system. Eleven members of the TOLL-like receptor have been identified in mammals. TLR 7 and TLR 9 are involved in the recognition of nucleic-acid-like structures. TLR 4 is involved in the recognition of polysaccharides.

The Pseudomonas Aeruginosa Hapten is compounded by three different elements: the polysaccharide itself, remains of nucleic acids (RNA or DNA) and a polypeptide or carrier. All three elements are believed to work as immunomodulator on the TOLL-like recognition system.

Strong expression of TLR's in prostate fibroblasts was found by Konig J.E. et al in BPH tissue.(The Prostate 2004; 58: 121- 129). Although more research would be needed regarding that, it seems obvious that TOLL-like receptor depending inflammatory cascades within the prostate tissue are crucial in the development of BPH.

According to the TOLL-like recognition system, PA polysaccharides are capable of blocking the direct stimulation of prostate fibroblasts by TLR 4, hereby inhibiting secretion of pro-inflammatory cytokines. At the same time, PAH is claimed to stimulate leukocyte migration and neutrophilic granulocytes resulting in increased re-absorption of necrotic and infarction focuses, often found in BPH tissue. Bacterial nucleic-acids recognition by TLR 9 is possibly involved in this matter.

Benign Hyperplasia of Prostate is one of the most common diseases affecting men and the number of cases increases year after year (Carter HB, Coffey DS Prostate; 16: 38 - 49, 1990).

Available treatment at present are: surgery, alpha-blockers or hormonal therapy (Caine M: J Urol 136: 1-4, 1986).

On the other hand, it is known that polysaccharides from some Gram negative bacterial wall are useful in the treatment of skin diseases and allergy.

An injected commercial product known as Poligram made out of bacterial polysaccharides it is used in dermatological treatments such as eczema, keloids, hypertrophy scars and ulcer.

EP1417969 to HPB SA discloses an injected liquid composition which includes polysaccharides from Gram negative bacteria, hydro-soluble extract of Thymus and hydro-soluble extract of Prostate. The disclosed way of application is long and complex, lasting 4 months. Subcutaneous injections of 1 mg are applied daily from day 0 up to 20; then 0.5 mg. from day 21 to 45, 6 days in a week (Sunday to rest); then 0.5 mg from day 46 to 66, 5 days a week; from day 67 till day 90: 0.5 mg 4 days in a week; and finally, 0.5 mg 3 days in a week (Monday, Wednesday and Friday) from day 91 up to day 120.

### BRIEF DESCRIPTION OF THE DRAWINGS

Figure 1 is a graphic showing the patient response to the treatment with the capsules of the invention, known in these figures as ORAL PAH. Parameters evaluation were performed on day Zero, 25 and 70 from the beginning of treatment. They were: Q max; post voided residual urine; digital rectal examination (DRE); prostate volume, Prostate specific antigen (PSA) and the original Boyarsky's IPSS (International Prostate Score System), as described in Example 5.
Figure 2 shows response of patients treated with the injected composition of the prior art. Parameters evaluation were done on days Zero, 45 and 120 of treatment. They were Q max. and post voided residual urine, as commented on Example 5.
Figure 3 shows response of patients treated with the injected composition of prior art. Parameters evaluation carried out during days zero, 45 and 120 of the trial, and were digital rectal examination (DRE) and the volume of prostate, as detailed on Example 5.
Figure 4 shows patient response treated with the injected composition of prior art. Parameters evaluations were done during days zero, 45 and 120 of the study, and they were: prostate specific antigen (PSA) and I-PSS, Boyarsky International Prostate Score System, as detailed on Example 5.

### SUMMARY OF THE INVENTION

An object of the present invention is to provide a solid pharmaceutical composition to treat Benign Prostate Hyperplasia, in which each unit (1 ml) of said composition contains at least between 0.5 and 5 mg, of polysaccharide extract of Gram negatives wall bacteria and pharmaceutical acceptable excipients. Said bacteria are preferably Pseudomonas Aeruginosa. Excipient are e.g. colloidal silicic anhydride, wheat starch, micro crystalline cellulose, lactose or a combination of both. The compound can be prepared as pastilles, capsules, tablets or pills. Preferably the composition is made as enteric capsules.

Another object of the present invention is to provide a method of preparation of the said solid composition for the treatment of Benign Prostate Hyperplasia which includes the following steps:
a) culturing bacteria Gram negative; preferably Pseudomonas Aeruginosa, in a nutritious agar broth;
b) extracting the lipo-polysaccharides from the bacterial walls and purify them;
c) separating the lipoid fraction in order to obtain polysaccharides in maximum purity;
d) drying the solution of polysaccharides and mill it; and
e) preparing a solid composition which at least includes the polysaccharides extract dried and milled in the previous step.

Another object of the present invention is to provide a method for the treatment of benign hyperplasia of prostate, that consists in providing to a needed individual a therapeutically effective quantity of a polysaccharide extract from a wall of a Gram negative bacteria. The quantity of the said polysaccharide extract may be between 0.5 mg and 5 mg per day, more preferably 1.5 mg per day. The administration of the composition of the invention is preferably oral.

### DETAILED DESCRIPTION OF THE INVENTION

The solid pharmaceutical composition of the present invention exceeds the prior art disclosed as injected compositions since it reduces the problem to the administration of one capsule of the invention per day which consist of 1.5 mg only during 70 days of treatment; so, the oral polysaccharide complex would be absorbed therefore acting in a more efficient way through out the intestinal mucous, very rich in Toll-like receptors. Moreover it is possible to reduce production costs; and finally common rejection to injections is avoided.

The solid pharmaceutical composition of the present invention includes an extract which contains polysaccharides of Gram negative bacteria and suitable excipient. The composition may be presented as tablets, capsules, pastilles and pills, being the capsules the preferred composition, prepared with cellulose acetoftalate and poly-ethyl-englicol 6000. Those skilled in the art know that it is possible to use polysaccharides extracted from the cellular wall of any Gram negative bacteria, being insomuch that all the polysaccharides from the cellular wall and the mixtures of both, are available to the present invention.

In the preparation of the composition of the invention is possible to employ any excipient, as long as the formula is resistant to the stomach pH and so, the active principle be liberated into the first intestinal portion.

In the essays performed to patients they were treated with a capsule per day of the composition of the invention, which capsule contained 1.5 mg of the polysaccharide extract from the cellular wall of the Pseudomonas bacteria. The placebo group received capsules containing only the excipient. Capsules for the treated group were prepared according to the disclosed method described in the examples detailed below. A total of 87 patients were included in the study, 44 were into the group treated with the composition of the invention and 43 patients were in the control group which received the placebo capsule.

We are hereby using the word PAH to define the group treated with the composition of the invention.

Results of the treatment are shown in Table 1

**- Table 1 - Clinical comparative response between the groups treated with the solid composition of the invention and Placebo**

| | PAH n=44 | PAH n=44 | PAH n=44 | Placebo n= 43 | Placebo n= 43 | Placebo n= 43 |
|---|---|---|---|---|---|---|
| | Day 0 | Day 25 | Day 70 | Day 0 | Day 25 | Day 70 |
| AGE | 66 ± 5 | | | | | |
| Q MAX. / ml. | 7.7 ± 45 | 10.8 ± 1.4 | 14.7 ± 1.9 | 6.0 ± 1.2 | 8.6 ± 3.6 | 15.7 ± 1.5 |
| RESIDUAL URINE/ml | 191 ± 48 | 90 ± 19.3 | 50. ± 10 | 175 ± 46 | 139 49.5 | ± 46 ± 16 |
| Digital Rectal Exam (DRE) (0 - 4) | 3.8 ± 0.7 | 2.5 ± 0.6 | 1.6 ± 0.4 | 3.7 ± 0.3 | 3.2 ± 0.9 | 1.6 ± 0.2 |
| Prostate/V olume ml | 69 ± 19 | 63 ± 18 | 55 ± 17 | 72 ± 22 | 77 ± 20.5 | 62 ± 17.7 |
| S.Prostate (SPA) Antigen | 7.1 ± 1.8 | 4.5 ± 2.2 | 3.1 ± 1.9 | 7.0 ± 1.1 | 5.9 ± 1.0 | 2.3 ± 0.9 |
| BOYARSKI SCORE (0-27) | 14.1 ± 0.2 | 7.3 ± 0.6 | 4.6 ± 0.4 | 14.1 ± 0.7 | 12.3 ± 0.5 | 7.6 ± 0.5 |

The patients treated with the solid composition of the invention showed clinical signs of improvement in the day 25 of the treatment, results being fairly better in comparison with those of the placebo group. Thereafter, as the placebo group began to be treated after day 25 with the solid composition of the invention, it is observed that on day 70 the group of control reaches clinical response similar to the PAH group initially treated from day zero of the assay.

On the other hand, the trial included comparative clinical studies employing the injected solution of the prior art. So, on Table 2 are described the results obtained with the injected solution in the PAH group of 119 patients in comparison with those 115 patients treated with placebo.

**- Table 2 - Clinical results between the groups treated with the Injected composition of the prior art and the Placebo**

| | PAH n=119 | PAH n=119 | PAH n=119 | Placebo n=115 | Placebo N=115 | Placebo n=115 |
|---|---|---|---|---|---|---|
| | DAY 0 | DAY 45 | DAY 120 | DAY 0 | DAY 45 | DAY 120 |
| AGE | 65 ± 8 | | | | | |
| Q MAX. /ml. | 8.1 ± 56 | 11.5 ± 2.1 | 15.5 ± 2.4 | 7.0 ± 1.4 | 8.3 ± 4.9 | 15.4 ± 2.2 |
| RESIDUAL /URINE ml. | 188 ± 56 | 89 ± 24.2 | 47.± 14 | 151 ± 45 | 148 58.1 | ± 45 ± 15 |
| Digital Rectal Exam. (DRE) (0-4) | 3.1 ± 0.9 | 2.3 ± 0.9 | 1.5 ± 0.6 | 3.4 ± 0.8 | 3.4 ± 0.8 | 1.5 ± 0.5 |
| Prostate Volume/ml. | 73 ± 24 | 65 ± 21 | 58 ± 18 | 73 ± 24 | 76 ± 23.1 | 63 ± 18.3 |
| Sp.Prostate Antigen (SPA) (ng) | 6.1 ± 2.9 | 4.9 ± 2.4 | 3.3 ± 2.0 | 6.4 ± 1.0 | 6.1 ± 1.1 | 2.5 ± 1.2 |
| BOYARSKI SCORE (0-27) | 13.5 ± 0.5 | 7.5 ± 0.5 | 5.5 ± 0.5 | 13.5 ± 0.5 | 14.5 ± 0.5 | 7.5 ± 0.5 |

As it can be observed only on day 45 those patients treated with the injected solution of the prior art achieved similar clinical response to the patients treated with the solid composition of the invention. We conclude therefore that the solid composition of the invention has early positive effects, in a period of time 40% shorter than the injected solution. The solid composition of the invention fulfill the treatment 70 days from day zero, while the injected solution needs 120 days of treatment. Therefore, the solid composition of the invention allows a shorten treatment and better accepted by the patient since said treatment is orally administered. Oral administration results to be an easily administered therapeutic way, particularly for extended and ambulatory treatments.

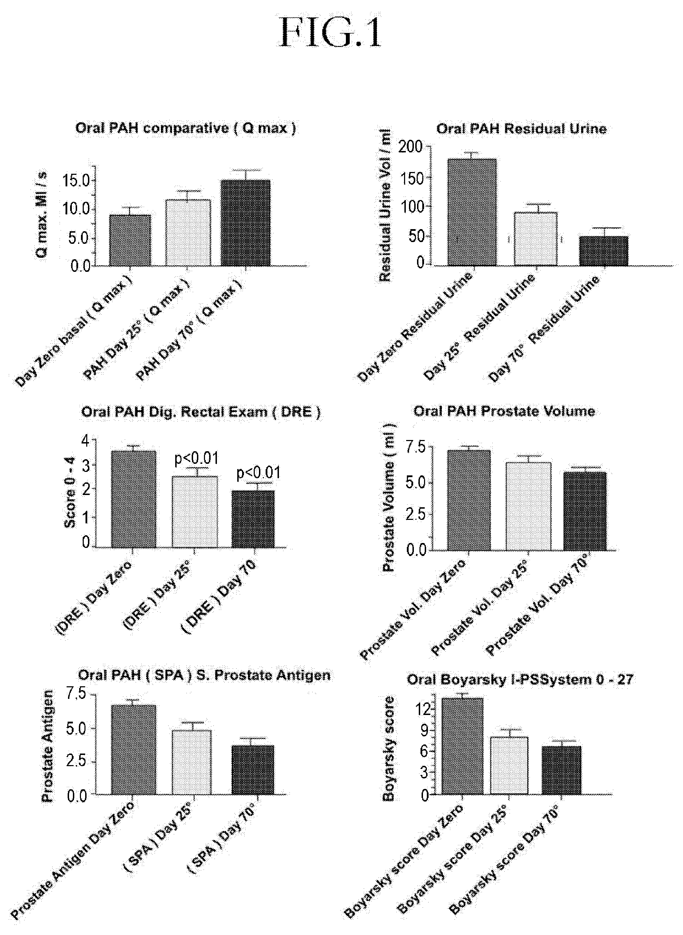

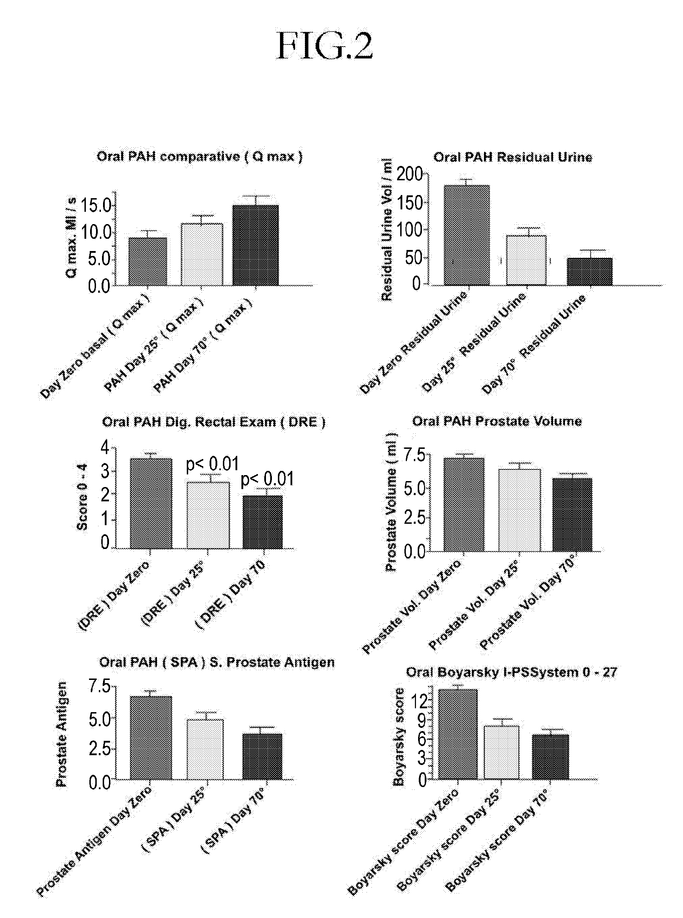

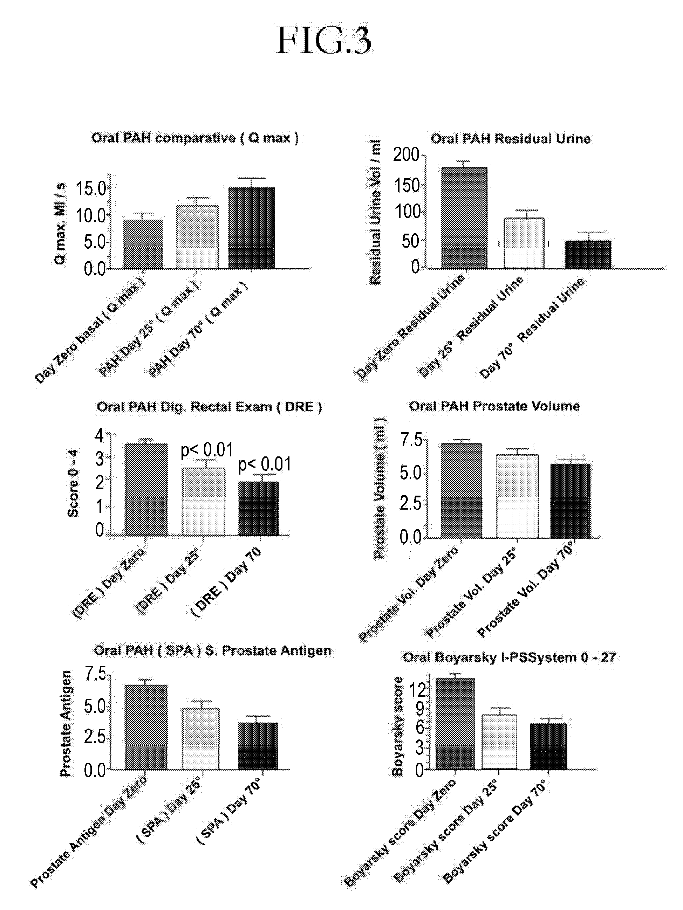

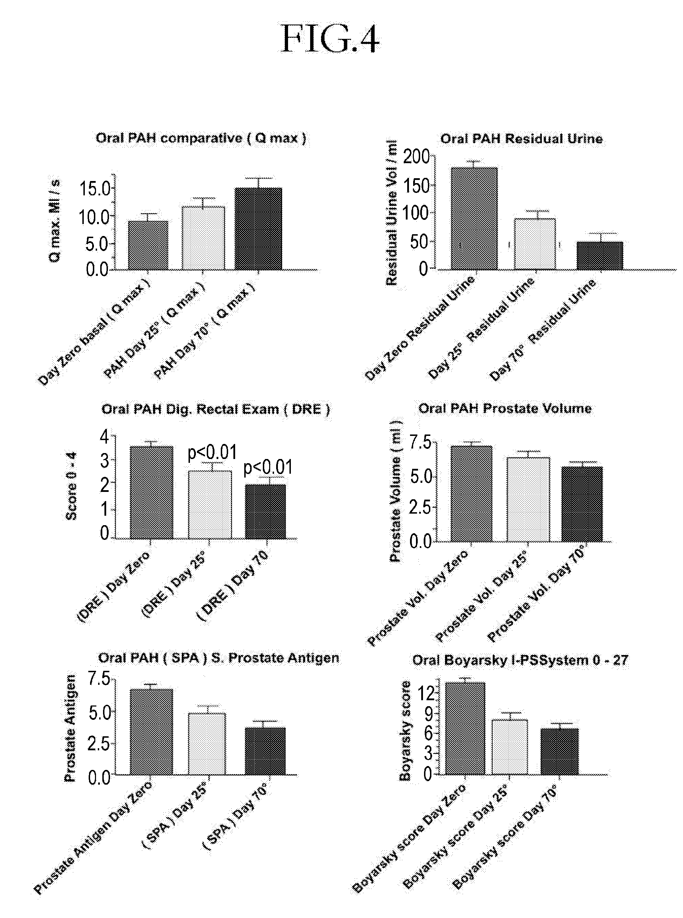

From the comparative analysis (see figures 1, 2, 3 and 4) it may be observed that the patients administered with the solid composition of the invention have a positive response to treatment 70 days from initiation, in all the evaluated parameters.

This invention is better explained according to the following Examples, which should not be taken just as a simple limitation to it. On the contrary, it must be clearly understood that it could be appealed to another performing, modification and equivalences of the same. And after reading through the present description of the invention those skilled in the art may be enlightened without departing from the spirit of the present invention and/or the scope of the appended claims.

### EXAMPLES

### Example 1: Culture of Pseudomonas bacteria.

Pseudomonas bacteria are sowed into Erlenmeyer jar containing a nutritious cultured broth, under sterility conditions. Growing of bacteria is allowed up to a logarithmic phase. Bacteria are centrifuged and kept frozen until its utilization.

For example, a concentrated cells suspension are sowed in a one liter capacity Erlenmeyer jar containing 250 cc of a liquid cultured medium.

As a common procedure, to obtain the bacterial growing curve, the count of bacteria versus time shall be put into graphics; for that purpose the commercial cultured medium must be prepared according to indications and five Erlenmeyer jars are filled with 250 cc each one. An sterilization procedure follows and when room-temperature is reached the cells stock are seeded and placed in agitator. Between a one hour period of time, measured exactly and on bacteriological condition, 1 mg of the culture is extracted then to proceed to the bacterial count. The bacterial counting is done through *the culture in a Petri plate, counting the number of developed colonies using nutritious Agar cultured medium, prepared as indicated. If it is necessary dilutions can be added.

Twice successively, 1 mg of the culture is extracted from each Erlenmeyer jar to proceed with the bacterial counting.

The bacterial growing curve is done with ten results, taking as the optimum culture time the moment the curve reaches the plateau-stage.
Once the optimum culture time is achieved, the bacterial mass production is started. For that purpose an specific number of Erlenmeyer jars are seeded in strict bacteriological conditions under a laminar flux bell. Then we proceed to weight.

Bacteria are kept under minus 20 centigrade until its final use.

It is understood that in all cases the corresponding bacteriological control of the stock will performed through specific biochemical tests in order to ensure they are bacteria free.

### Example 2: Procedure for the extraction of lipo polysaccharides of Pseudomonas

Bacteria are cultured in nutritious agar during 24 hours.
Sodium chloride 0.15M, is added to the culture, turned into suspension and centrifuged during 15 minutes to 5,000 rpm. Three washings with acetone are performed and the resulting microbial paste is thinly spread and put to dry. The bacterial dried paste is grinded in a ball-mill and the resulting powder is put through a sieve.

10 gr of the previously obtained powder is put on suspension in 175 ml of water to 65-68°C; an equal volume of phenol at 90% previously heated at same temperature is added, then vigorously agitated. The mixture is kept to 65°C during 10 to 15 minutes; placed in an iced-bath to cold at 10°C and centrifuged at 3,000 rpm during 45 minutes. It is obtained an aqueous layer, another phenolic and a third one of unsolvable leftovers. The aqueous layer is separated and kept apart. The phenol layer and the unsolvable leftover are extracted again with 175 ml of hot water, as previously explained. Aqueous extracts are mixed and dialyzed with distilled water in order to eliminate rests of phenol and low molecular weight substances of bacterial origin. Under lower pressure, the opalescent solution contained into the polysaccharide and the ribonucleic acid, is then concentrated to a temperature of 35-40°C until a volume of 50 ml is reached; then is centrifuged in order to discard unsolvable remnants. The floating material must be lyophilized.

The lyophilized material is dissolved into distilled water and in a proportion of 150 ml. for each gr. of remnant we add 15 ml of cetyl tri methyl ammonium bromide (Cetavion), 2 % in water.
The mixture is to be agitated at room-temperature during 15 minutes and then centrifuged 20 minutes at 3,000 rpm in order to discard the precipitated RNA. Floating material is to be lyophilized and then be dissolved again into 50-60 ml of sodium chloride 0.5 M. Finally, lipo-polysaccharides are precipitated by adding five ethanol volume. Placed at 4°C during 2 hours, it is centrifuged and dissolved again into a convenient volume of distilled water.

The lipo-polysaccharide that is obtained represents approximately 2 % of the dry weight of processed bacteria.

### Example 3: Procedure to obtain polysaccharides

From the lipo-polysaccharide obtained in the previous phase, a (10mg/ml) solution in acetic acid is prepared at 1%; it is heated at 100 C during 30 minutes in order to produce hydrolysis and then centrifuged at 500 g to separate the unsolvable lipid A. The floating material is dialyzed with distilled water at 4 °C during 48 hours and finally the obtained product is lyophilized. As a result of this process the obtained product is a polysaccharide complex compounded by three elements: a) the polysaccharide itself; b) remnants of nucleic acid (DNA & RNA); and c) amino acids and low molecular weight polypeptides. Through the TLR (Toll- like- Receptors),polysaccharides and particularly the nucleic acids, would work through immune-modulators mechanisms, according to description in the precedent bibliography.

### Example 4: Preparation of the solid pharmaceutical composition of the invention

A concentrated solution of 5 mg/ml from the polysaccharide is prepared. Solution is put to dry with silicic anhydride, then the following excipients are added: Colloidal silicic anhydride, wheat starch, micro crystalline cellulose and lactose. Enteric coverage is done with cellulose acetophtalate and Poly ethilenglycol 6,000.

Mixture is homogenized and then encapsulated. The procedure ends with the enteric covering.

### Example 5: Clinical research with the pharmaceutical composition of the invention in patients with benign prostate hyperplasia (BPH)

One hundred twenty six patients with BPH and urine problems participated at the clinical essay.

Exclusion criteria: prostate cancer, urinary stasis leading to renal failure; Detrusor instability or incompensation; cerebro-vascular illness; severe neurological or psychiatric disorders; urethral post- traumatic stricture; large diverticulum of the bladder; bladder calculi; neurological bladder; previous prostate surgery; chronic prostatitis or other disorder post invasive procedure.

Inclusion criteria: all patients with BPH symptoms were evaluated according to the (I-PSS) International Prostate Score System Boyarsky (0-27 scoring); urodynamic testing (Urobite 1000, Promedon 1997); post micturition residual volume( assessed by Ultrasound; Specific prostate antigen (SPA). In order to rule out prostate cancer, in all cases with SPA > than 4 ug / ml a biopsy was performed.

Prostate volume was monitored by trans-rectal ultrasound and a digital-rectal examination (DRE) to evaluate consistency and hardness of the prostate gland. This parameter was divided into 4 degrees: 1^{st}. degree = very soft; 2^{nd}. degree = soft; 3^{rd}. degree = hard and 4^{th}.degree = very hard. All participants with 4^{th} degree consistency were performed prostate biopsy .

Randomization: Patients were divided in two groups: the active group received the solid composition of the invention and the control group treated with placebo. After a detailed explanation of the research program to the candidates, the informed consent was signed.
Eighty seven candidates were finally accepted in the double- blind, randomized and placebo controlled study. The active group (44 patients) were treated with the composition of the invention without interruption during 70 days (until day 70 ) in a single dose of 1,5 mg per day. Control group (43 patients)received instead placebo capsules, from day zero to day 25 . After that, (from day 26 ahead) began the treatment with capsules 1.5 mg, daily dose of the composition of the invention until a full period of 70 days was completed. Meaning that both groups (active and control groups as well) were treated during 70 days with the composition of the present invention: a daily capsule of 1.5 mg each. Thereafter all tests were concluded.

All laboratory proofs, Boyarsky I-PSS and additional tests - done on Day Zero - were repeated on day 25 and day 70 in every patient of both groups.

## Claims

1. A solid pharmaceutical composition for the treatment of Benign Prostate Hyperplasia **characterized in that** each Unit contains at least between 0.5 and 5 mg. of an extract of polysaccharide of the Gram negative bacteria wall together with pharmaceutically acceptable excipients.

2. The composition as claimed in claim 1, wherein excipients are selected from the group consisting of colloidal silicic anhydride, wheat starch, microcrystalline cellulose, lactose and a combination thereof.

3. The composition as claimed in claim 1, wherein said solid composition is selected from the group consisting of pastilles, capsules, tablets and pills.

4. The composition as claimed in claim 1, wherein the extract further includes nucleic acids and polypeptides.

5. The composition as claimed in claim 1, wherein the bacteria is *Pseudomonas Aeruginosa.*

6. A method for preparing a solid composition for the treatment of Benign Prostate Hyperplasia as claimed in claim 1, wherein it comprises the steps of:
a) culturing in a nutritive agar medium Gram negative bacteria;
b) extracting lipo-polysaccharides from the said bacteria wall and purified it;
c) separating the lipid fraction in order to obtain the purified polysaccharides;
d) drying the obtained polysaccharides solution , then mill it; and
e) preparing a solid composition which at least includes the polysaccharide extract dried and milled in the previous phase.

7. The method as claimed in claim 6, wherein the Gram negative bacteria is *Pseudomonas Aeruginosa.*

8. The method as claimed in claim 6, wherein the solid composition prepared in step e) is a selected composition of the group comprising tablets, capsules, pastilles and pills.

9. The method as claimed in claim 6, wherein pharmaceutically acceptable excipients are further added in step e).

10. Use of a therapeutically effective amount of an extract of a Gram negative bacteria wall polysaccharide for the manufacture of a medicament for the treatment of benign prostate hyperplasia.

11. The use as claimed in claim 10, wherein the therapeutically effective amount is ranging between 0.5 and 5 mg per day.

12. The use as claimed in claim 11, wherein the therapeutically effective amount is 1.5 mg per day.

13. The use as claimed in claim 10, wherein the extract of polysaccharides is in a solid form.

14. The use as claimed in claim 13, wherein the solid form is selected from the group consisting of tablets, capsules, pastilles and pills.

15. The use as claimed in claim 14, wherein the solid form is an enteric coated capsule.

16. The use as claimed in claim 10, wherein the Gram negative bacteria is *Pseudomonas Aeruginosa.*

17. The use as claimed in claim 10, wherein the polysaccharide extract further includes nucleic acids, amino acids and polypeptides.

18. The use as claimed in claim 10, wherein it is administered orally.
